(19) Europäisches Patentamt
European Patent Office
Office européen des brevets

(11) Numéro de publication: **0 057 337**
**B1**

(12) # FASCICULE DE BREVET EUROPEEN

(45) Date de publication du fascicule du brevet: **14.03.84**

(21) Numéro de dépôt: **81401752.1**

(22) Date de dépôt: **30.10.81**

(51) Int. Cl.[3]: **C 07 D 307/93,** C 07 D 491/06, A 61 K 31/34, A 61 K 31/50 // C07D307/54, C07D307/36, C07D307/42, C07D307/68, C07D493/08 ,(C07D491/06, 307/00, 237/00)

(54) **Dérivés du 4H-benzo(4,5)cyclohepta(1,2-b)furanne, leur procédé de préparation et leur application en tant que médicaments.**

(30) Priorité: **10.11.80 FR 8023973**

(43) Date de publication de la demande: **11.08.82 Bulletin 82/32**

(45) Mention de la délivrance du brevet: **14.03.84 Bulletin 84/11**

(84) Etats contractants désignés: **AT BE CH DE FR GB IT LI LU NL SE**

(56) Documents cités: **FR - A - 2 344 284**

(73) Titulaire: **Delbarre, Bernard, Ferme "Les Carnaux" Ballan Mire, F-37300 Joue-les-Tours (FR)**
Titulaire: **Mavoungou-Gomes, Louis, 3, rue Desportes, F-49000 Angers (FR)**

(72) Inventeur: **Delbarre, Bernard, Ferme "Les Carnaux" Ballan Mire, F-37300 Joue-les-Tours (FR)**
Inventeur: **Mavoungou-Gomes, Louis, 3, rue Desportes, F-49000 Angers (FR)**

(74) Mandataire: **Rinuy, Guy et al, 14, Avenue de la Grande Armée, F-75017 Paris (FR)**

**EP 0 057 337 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Dérivés du 4H-benzo [4,5] cyclohepta [1,2-b] furanne, leur procédé de préparation et leur application en tant que médicaments

La présente invention a pour objet des dérivés du 4H-benzo-[4,5]-cyclohepta [1,2-b] furanne, un procédé permettant de les préparer et leurs applications notamment en thérapeutique.

Les composés qui font l'objet de la présente invention répondent à la formule:

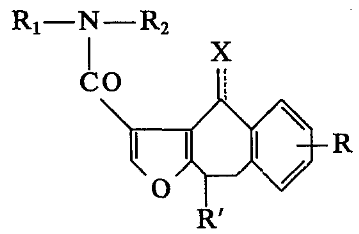

(I)

dans laquelle R représente un atome d'hydrogène, un radical alcoyle ayant 1 à 6 atomes de carbone ou un radical alkoxy ayant de 1 à 6 atomes de carbone,

R' représente un atome d'hydrogène, un radical alcoyle ayant de 1 à 6 atomes de carbone, un radical phényle ou un radical phénylalcoyle dont la partie alcoyle a de 1 à 6 atomes de carbone,

X est un radical hydroxy ou un radical oxo et $R_1$ et $R_2$, pris séparément, représentent, indépendamment l'un de l'autre, un atome d'hydrogène, un radical alcoyle ayant de 1 à 6 atomes de carbone portant éventuellement un radical cyano ou un radical de formule:

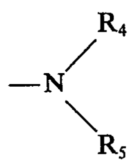

dans laquelle $R_4$ et $R_5$ représentent, indépendamment l'un de l'autre, un atome d'hydrogène, un radical alcoyle ayant de 1 à 6 atomes de carbone ou forment ensemble et avec l'atome d'azote sur lequel ils sont fixés un radical morpholino,

et $R_1$ et $R_2$, ensemble, forment avec l'atome d'azote sur lequel ils sont fixés, un radical morpholino, pipéridino ou pipérazino portant éventuellement sur le deuxième atome d'azote un radical alcoyle ayant de 1 à 6 atomes de carbone ou un radical phénylalcoyle dont la partie alcoyle a de 1 à 6 atomes de carbone,

ou bien X est un reste aminal

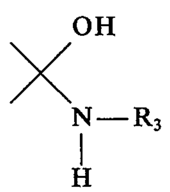

ou un radical de formule $= N - R_3$, $R_3$ représentant un atome d'hydrogène ou un radical alcoyle ayant de 1 à 6 atomes de carbone portant éventuellement un radical de formule:

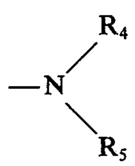

dans laquelle $R_4$ et $R_5$ ont la même signification que celle donnée précédemment, $R_1$ est identique à $R_3$ et $R_2$ est un atome d'hydrogène,

ou bien X forme avec $R_2$ un pont $= N -$ et $R_1$ représente un atome d'hydrogène ou un radical alcoyle ayant de 1 à 6 atomes de carbone portant éventuellement un radical de formule:

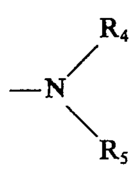

dans laquelle $R_4$ et $R_5$ ont la signification donnée précédemment.

La présente invention a également pour objet les sels d'addition des composés de formule (I) avec des acides.

Les composés qui font l'objet de la présente invention peuvent être préparés selon le schéma réactionnel suivant:

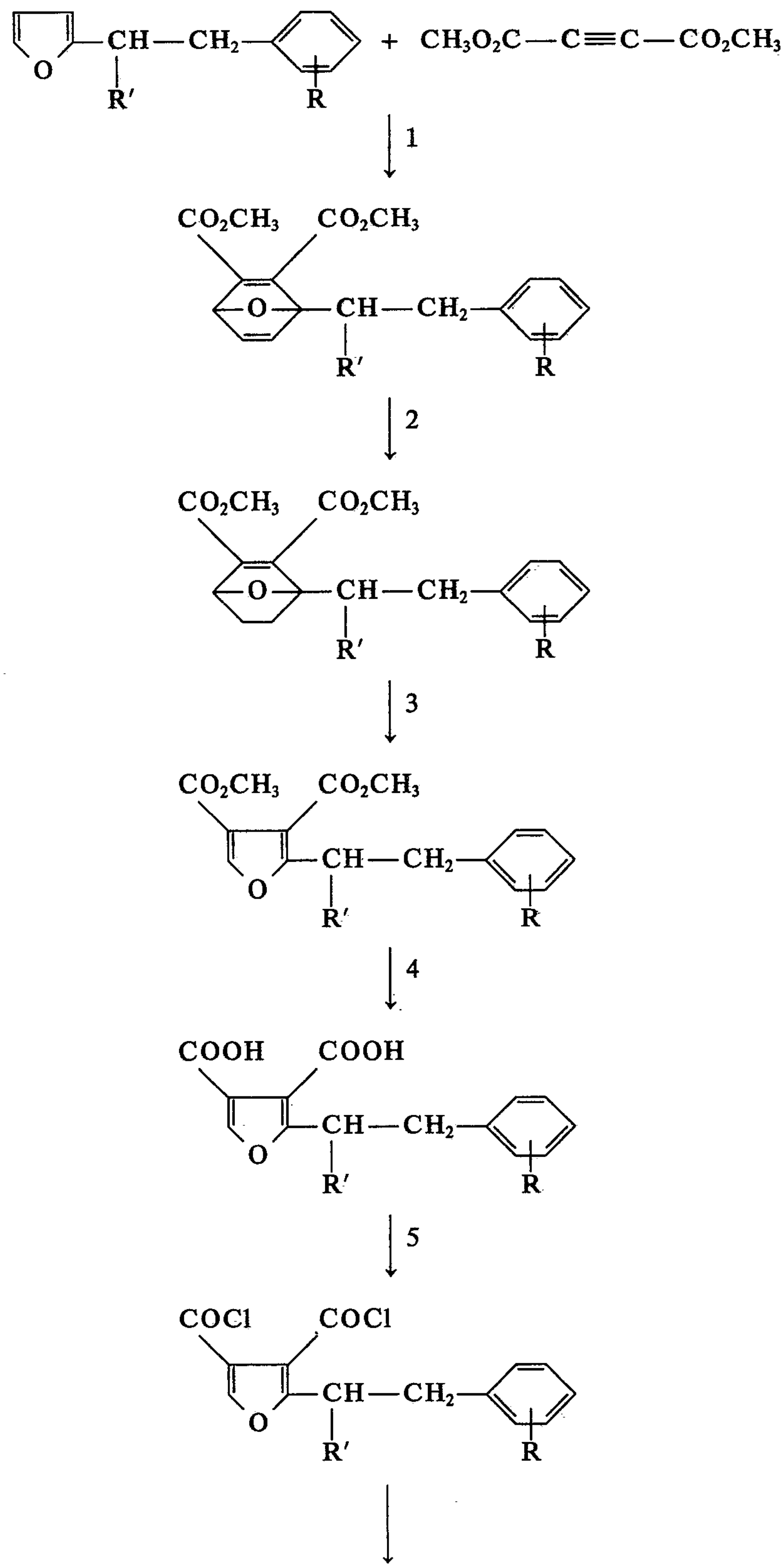


(II)

(III)

(IV)

(V)

(VI)

(VII)

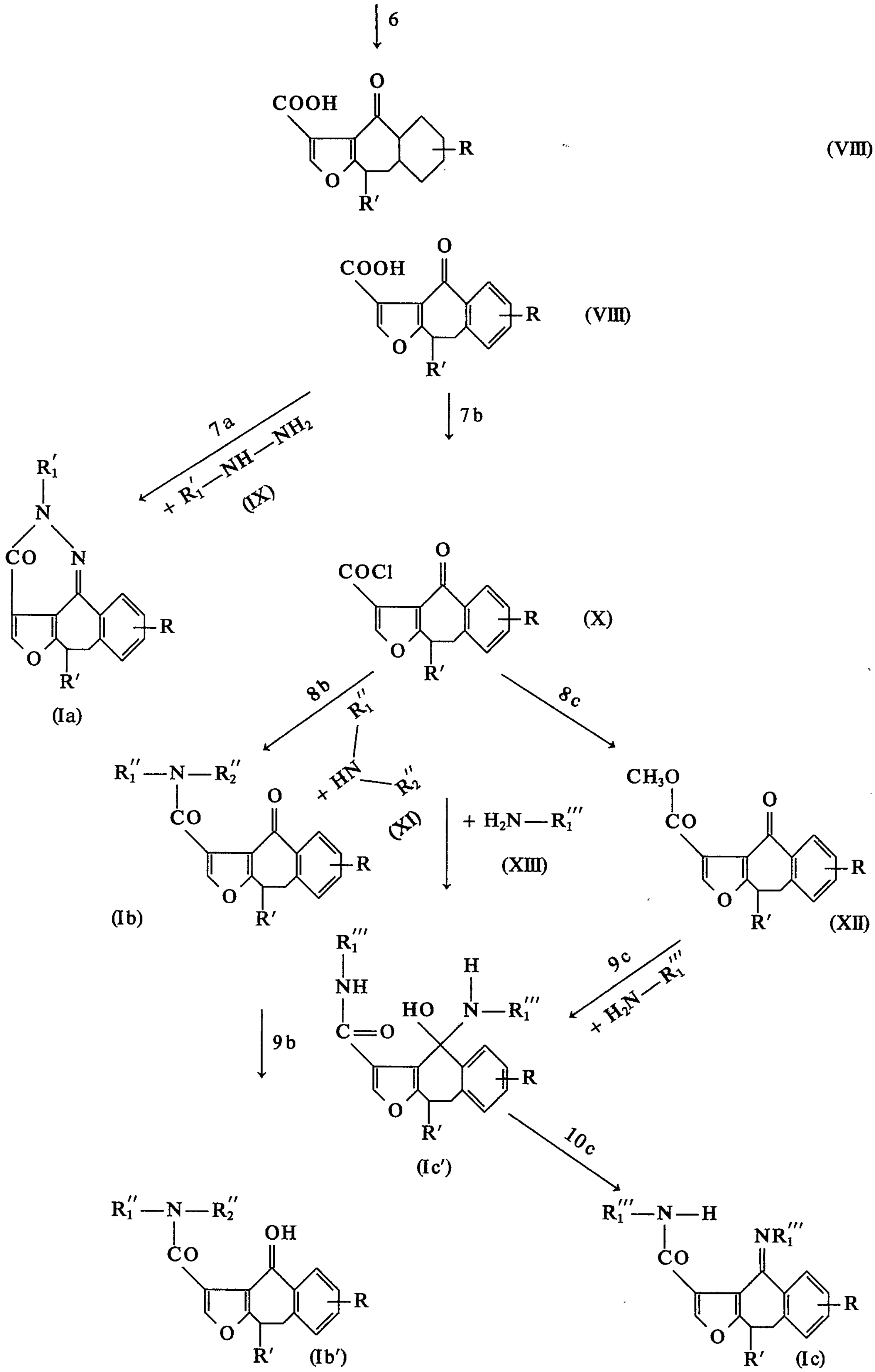


(VIII)

(VIII)

(Ia)

(X)

(Ib)

(XII)

(Ic')

(Ib')

(Ic)

Dans ce schéma R et R′ ont la même signification que dans la formule (I).

$R_1'$, $R_1''$ et $R_2'''$ ainsi que $R_1''$, ont les significations données pour $R_1$, $R_2$ et $R_3$ respectivement dans les cas où X forme avec $R_2$ un pont, X est un groupe oxo ou hydroxyle et X est un groupe

$=N-R_3$ ou 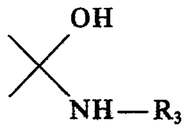

Les différents stades de synthèse sont les suivants:

Stade 1:

On effectue une condensation (synthèse diénique) d'une ($\beta$-aryl) alkyl-2 furanne (II) avec l'acétylène dicarboxylate de méthyle dans un solvant tel que le benzène. On obtient un dérivé de l'oxa-7 bicyclo [2,2,1] heptadiène-2,5 (III) qui n'est pas isolé.

Stade 2:

On effectue une hydrogénation partielle du composé de formule (III) notamment dans de l'éthanol en présence de charbon palladié à 10% ou dans de l'acétate d'éthyle en présence de nickel de Raney. On obtient le composé de formule (IV).

Stade 3:

Ce composé de formule (IV) est transformé directement selon une réaction d'Alder Rickert en ($\beta$-aryl)-alkyl-2 dicarbométhoxy-3,4 furanne (V).

Stade 4:

Par saponification du diester (V), on obtient le diacide (VI) correspondant.

Stade 5:

Le diacide est transformé en dichlorure (VII) par action du chlorure de thionyle.

Stade 6:

On effectue une cyclisation intramoléculaire du dichlorure (VII) en solution dans le sulfure de carbone en présence de chlorure d'aluminium. Le traitement aqueux du produit réactionnel donne un céto-acide de formule (VIII).

En variante, le céto-acide (VIII) peut également être obtenu par cyclisation directe du diacide (VI) dans de l'acide polyphosphorique entre 100 et 130° C.

Stade 7a:

Le traitement du céto-acide (VIII) par une hydrazine (IX) conduit à un composé de formule (I) dans laquelle X forme avec $R_2$ un pont $= N-$ (formule Ia).

Stade 7b:

Le traitement du céto-acide (VIII) par le chlorure de thionyle conduit au chlorure d'acide (X).

Stade 8b:

Le traitement du chlorure d'acide (X) par une amine (XI) conduit à un composé de formule (I) dans laquelle X est un radical oxo (formule 1b).

Stade 9b:

La réduction du composé (Ib) par $BH_4K$ conduit au composé de formule (I) dans laquelle X est un radical hydroxyle (formule I′b).

Stade 8c:

Le traitement du chlorure d'acide (X) par le méthanol conduit à l'ester (XII).

Stade 9c:

Le traitement de l'ester (XII) par une amine (XIII) conduit au composé Ic′; ce produit peut être également obtenu en traitant le chlorure d'acide (XI) par l'amine (XIII) en excès. Le dérivé Ic′ par déshydratation conduit au composé de formule (I) dans laquelle X est un radical $= N-R_3$ (formule Ic) (stade 10c).

Certains des composés de formule (II) utilisés comme matière de départ pour la synthèse des composés de formule (I) sont sonnus (Freund, Immerwahr Ber. 1890, 23, 2847 et R. B. Woodward J. Amer. Chem. Soc. 1940, 62, 1478).

Les procédés de préparation décrits n'ont pas donné toutefois de résultats satisfaisants. Aussi ces composés ont été préparés selon un procédé original dont le schéma réactionnel est le suivant:

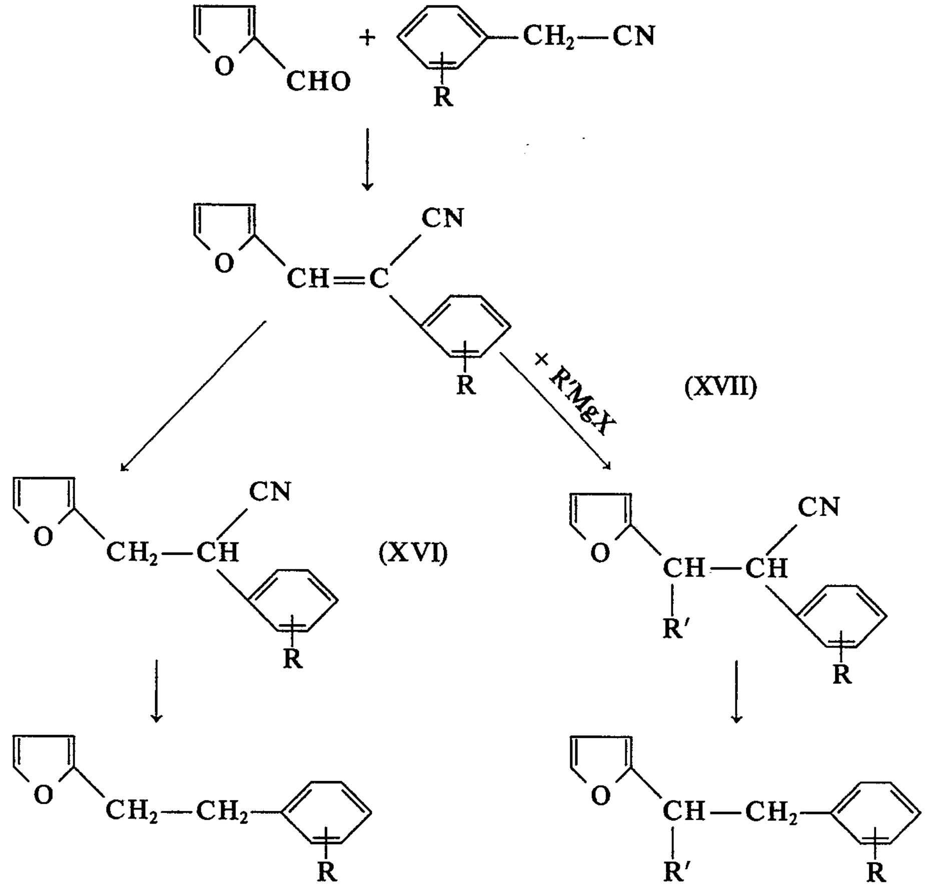


(XIV)

(XV)

(XVII)

(XVI)

(XVIII)

(IIa)

(IIb)

Ce procédé consiste à condenser tout d'abord le furfural avec un arylacétonitrile (XIV) en présence d'éthylate de sodium.

On obtient ainsi un aryl-2 (α-furyl)-3 propènenitrile (XV) avec un bon rendement.

Pour préparer les composés de formule (II) dans laquelle R' est un atome d'hydrogène (formule IIa), on effectue une hydrogénation à l'aide du borohydrure de potassium en solution hydro-alcoolique. Le nitrile saturé obtenu (XVI) est alors soumis à une scission réductive par le sodium et l'éthanol. Pour préparer les composés de formule (II) dans laquelle R' est différent de l'hydrogène (formule IIb) on effectue une addition de l'organomagnésien (XVII) sur le nitrile (XV) et on soumet le nitrile saturé obtenu à une scission réductive par le sodium et l'éthanol.

Les exemples suivants illustrent la préparation des composés qui font l'objet de l'invention.

A. — Preparation des composes de depart

(a) Phényl-2 (α-furyl)-3 propènenitrile

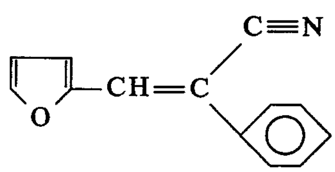

Dans un ballon de six litres muni d'un agitateur mécanique, d'une ampoule à brome et d'un réfrigérant, on introduit 5 moles de furfural fraîchement distillé et 5,10 moles de cyanure de benzyle. Le ballon étant refroidi sur glace, sous agitation, on ajoute par l'ampoule à brome une solution éthanolique d'éthylate de sodium préparée à partir de 23 g de sodium et 400 $cm^3$ d'éthanol pur. La réaction est exothermique. Cinq minutes après l'addition de l'éthylate, on ajoute 65 g d'acide acétique, 2,5 litres de benzène et 1 litre d'eau. La phase organique est décantée, lavée à l'eau, et séchée sur sulfate de sodium. Après élimination du benzène, le résidu est distillé sous pression réduite.

| | |
|---|---|
| Rdt minimum | 95% |
| E/15 mm Hg | 190 – 195° C |
| F | 41 – 42° C |

Cristaux jaunâtres brunissant à l'air.

(b) Phényl-2 ($\alpha$-furyl)-3 propanenitrile

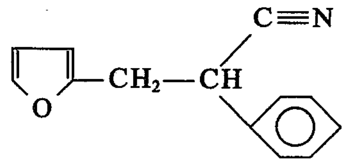

Dans un ballon de 2 litres à 3 tubulures, muni d'un agitateur mécanique et d'un réfrigérant, on dissout à chaud 200 g de phényl-2 ($\alpha$-furyl)-3 propènenitrile dans 300 $cm^3$ d'éthanol absolu. La solution refroidie est additionnée de 30 g de borohydrure de potassium finement pulvérisé, puis de 100 $cm^3$ d'eau. Le mélange est porté au reflux pendant 7 heures, puis abandonné 12 heures à température ambiante. Après acidification par 50 $cm^3$ d'acide acétique, l'éthanol est distillé. Le résidu se sépare en deux couches. On extrait au benzène (500 $cm^3$). La solution benzénique est lavée à l'eau, séchée sur sulfate de sodium. Après élimination du benzène, le nitrile saturé est distillé sous pression réduite.

| | |
|---|---|
| Rendement | 170 g, 85% |
| E/13 mm Hg | 167 – 168° C |
| $n_D{}^{20}_{19}$ | 1,5444 |
| $d_4$ | 1,090 |

(c) Phénéthyl-2 furanne

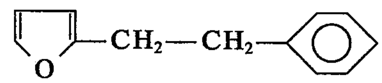

Dans un ballon de deux litres à trois tubulures, muni d'un agitateur, d'un réfrigérant et d'une ampoule à brome, on disperse à chaud 37 g de sodium dans 175 $cm^3$ de toluène bouillant. Le chauffage étant arrêté et l'agitation maintenue, on ajoute par l'ampoule à brome 0,25 Mole de phényl-2 ($\alpha$-furyl)-3 propanenitrile mélangé à 40 $cm^3$ d'éthanol absolu. L'addition est réglée de manière à maintenir un reflux auto-entretenu du mélange réactionnel. On ajoute de l'éthanol goutte à goutte de façon à détruire le sodium en excès. Le mélange est ensuite refroidi, traité avec un litre d'eau. Il se sépare en deux couches. La couche organique est séparée; la couche aqueuse est saturée de chlorure de sodium et extraite au toluène (400 $cm^3$). Les solutions organiques sont mélangées, lavées à l'eau et séchées sur sulfate de sodium. Après distillation du toluène, le résidu est rectifié sous pression réduite.

| | |
|---|---|
| Rendement | 90% |
| E/10 mm Hg | 112°C |
| $n_D{}^{19}_{19}$ | 1,5385 |
| $d_4$ | 1,023 |

On a rassemblé ci-dessous les caractéristiques des composés de formule (II) préparés par scission réductive des nitriles saturés correspondants.

| R | R′ | E/mm | $n_D^{19}$ | $d_4^{19}$ | Rdt (%) | Formule brute | C (%) Cal. | C (%) tr. | H (%) Cal. | H (%) tr. |
|---|---|---|---|---|---|---|---|---|---|---|
| H | H | $112^{\circ}_{10}$ | 1,5385 | 1,023 | 90 | $C_{12}H_{12}O$ | 83,73 | 83,70 | 6,97 | 7,1 |
| H | $CH_3$ | $116^{\circ}_{12}$ | 1,5315 | 1,005 | 88 | $C_{13}H_{14}O$ | 83,88 | 83,80 | 7,52 | 7,1 |
| H | $C_2H_5$ | $125^{\circ}_{11}$ | 1,5247 | 0,995 | 90 | $C_{14}H_{16}O$ | 84,00 | 83,75 | 8,00 | 8,3 |
| H | n-$C_3H_7$ | $136^{\circ}_{12}$ | 1,5200 | 0,981 | 85 | $C_{15}H_{18}O$ | 84,12 | 83,90 | 8,39 | 8,5 |
| H | n-$C_4H_9$ | $146^{\circ}_{12}$ | 1,5158 | 0,966 | 83 | $C_{16}H_{20}O$ | 84,22 | 83,90 | 8,77 | 9,0 |
| H | n-$C_5H_{11}$ | $156^{\circ}_{12}$ | 1,5123 | 0,968 | 85 | $C_{17}H_{22}O$ | 84,31 | 84,30 | 9,08 | 9,1 |
| H | $C_6H_5$ | $191^{\circ}_{14}$ | 1,5780 | 1,074 | 80 | $C_{18}H_{16}O$ | 87,10 | 86,50 | 6,44 | 6,4 |
| H | $CH_2-C_6H_5$ | $194^{\circ}_{12}$ | 1,5695 | 1,062 | 87 | $C_{19}H_{18}O$ | 87,03 | 87,00 | 6,86 | 7,1 |
| p · $OCH_3$ | H | $156^{\circ}_{14}$ | 1,5420 | 1,074 | 60 | $C_{13}H_{14}O_2$ | 77,24 | 77,25 | 6,92 | 7,0 |

B. — Preparation des composes selon l'invention

(1) Phénéthyl-2 dicarbométhoxy-3,4 furanne

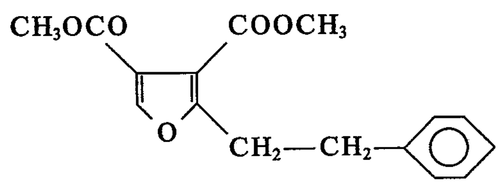

(a) Synthèse diénique

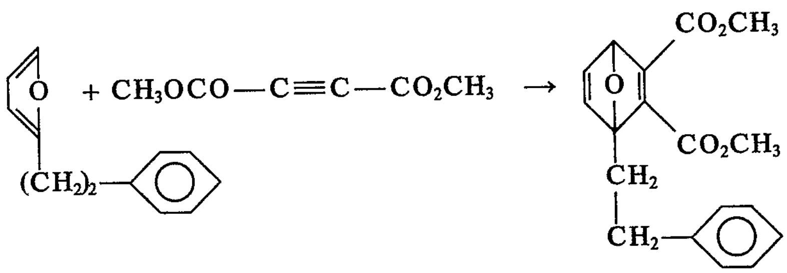

Dans un ballon de 1 litre, on mélange 0,5 mole de phénéthyle-2 furanne, 71 g d'acétylènedicarboxylate de méthyle et 120 cm³ de benzène. Le mélange réactionnel est porté au reflux pendant 6 heures. Au bout de ce temps, le caractère lacrymogène du diester acétylique a pratiquement disparu. Le benzène est éliminé sous pression réduite; le résidu du ballon est additionné de 300 cm³ d'acétate d'éthyle.

(b) Hydrogénation partielle

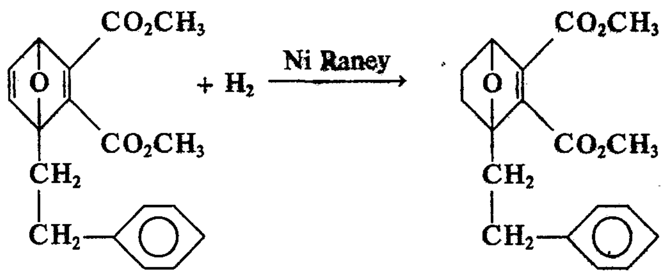


La solution dans l'acétate d'éthyle obtenue au stade précédent est hydrogénée en présence de 10 g de nickel de Raney préalablement lavé à l'eau (trois fois), à l'éthanol (trois fois) et à l'acétate d'éthyle (trois fois), ce lavage rigoureux permettant une meilleure dispersion du catalyseur dans le solvant utilisé. La réaction est exothermique, et dure au plus 45 minutes à 1 heure (fixation de 11,2 à 11,4 litres d'hydrogène). La solution est filtrée; le filtrat est évaporé sous pression réduite. Le résidu visqueux et brunâtre est introduit dans un ballon de Vigreux de 250 $cm^3$ monté pour une distillation sous pression réduite (tube capillaire, thermomètre).

(c) Pyrolyse

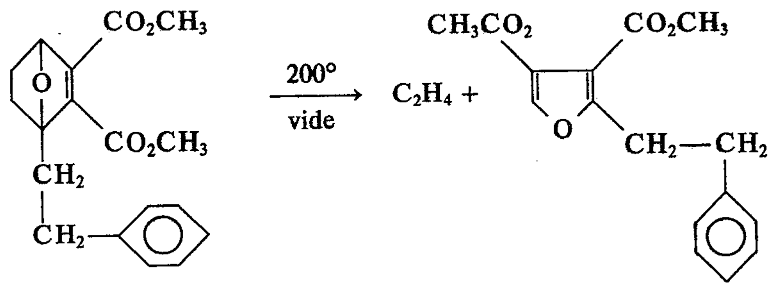


Le ballon précédent est relié à un réfrigérant, un séparateur et au vide d'une trompe à eau; la pression initiale est de 12 à 15 mm de mercure. Au cours du chauffage, la thermolyse de l'hydrogénat partiel dégage de l'éthylène qui s'élimine dans la trompe à eau; il y a augmentation de la pression intérieure, celle-ci ne revenant à sa valeur initiale que lorsque la pyrolyse est terminée. On distille alors le résidu du ballon en prenant soin d'éviter la surchauffe.

On a rassemblé dans le tableau ci-dessous les caractéristiques du composé ainsi préparé ainsi que celles d'autres composés de formule (V) préparés de manière analogue.

| R | R' | E/mm | $n_D^{19}$ | $d_4^{19}$ | Rdt (%) | Formule brute | C (%) Cal. | C (%) tr. | H (%) Cal. | H (%) tr. |
|---|---|---|---|---|---|---|---|---|---|---|
| H | H | $212^{\circ}_{12}$ | 1,5335 | 1,179 | 91 | $C_{16}H_{16}O_5$ | 66,68 | 66,60 | 5,55 | 5,60 |
| H | $CH_3$ | $219^{\circ}_{19}$ | 1,5290 | 1,166 | 84 | $C_{17}H_{18}O_5$ | 67,55 | 67,10 | 5,96 | 6,55 |
| H | $C_2H_5$ | $221^{\circ}_{19}$ | 1,5250 | 1,153 | 83 | $C_{18}H_{20}O_5$ | 68,35 | 67,90 | 6,33 | 6,45 |
| p · $OCH_3$ | H | $250^{\circ}_{20}$ | 1,5370 | 1,204 | 88 | $C_{17}H_{18}O_6$ | 64,15 | 63,30 | 5,66 | 5,70 |

(2) Phénéthyl-2 dicarboxy-3,4 furanne

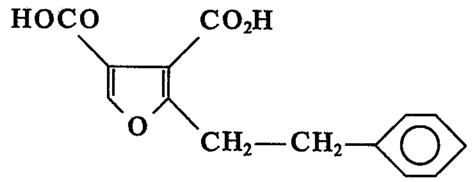

On porte au reflux pendant 5 heures un mélange de 0,2 M de phénéthyl-2 dicarbométhoxy-3,4 furanne, 56 g de potasse, 200 cm³ d'éthanol et 70 cm³ d'eau. L'éthanol est ensuite distillé à pression ambinate. Le résidu est additonné de 200 cm³ d'eau, refroidi et versé sur 100 cm³ d'acide chlorhydrique pur (environ 12 N). Le précipité est lavé à l'eau, séché. Il est recristallisé dans le mélange éthanol/eau.

On a rassemblé dans le tableau ci-dessous les caractéristiques du composé ainsi préparé ainsi que celles d'autres composés de formule (VI) préparés de manière analogue.

| R | R' | F°C | Rendement % | Formule brute | C % Cal. | C % tr. | H % Cal. | H % tr. |
|---|---|---|---|---|---|---|---|---|
| H | H | 148° | 98 | $C_{14}H_{12}O_5$ | 64,61 | 65,05 | 4,61 | 4,63 |
| H | $CH_3$ | 207° | 98 | $C_{15}H_{14}O_5$ | 65,70 | 65,35 | 5,10 | 4,95 |
| H | $C_2H_5$ | 167° | 98 | $C_{16}H_{16}O_5$ | 66,66 | 66,00 | 5,55 | 5,25 |
| p · $OCH_3$ | H | 165° | 97 | $C_{15}H_{14}O_6$ | 62,07 | 62,20 | 4,82 | 4,73 |

(3) Carboxy-3 one-4 4H-benzo [4,5] cyclohepta-[1,2-b) furanne

(a)

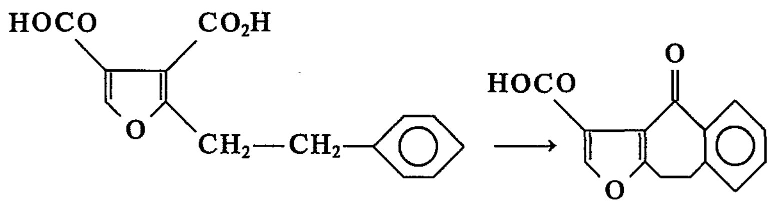

26 g de phénéthyl-2 dicarboxy-3,4 furanne brut et sec sont finement pulvérisés et mélangés à 200 g d'acide polyphosphorique commercial. Le mélange est agité et chauffé pendant 30 à 40 minutes entre 100 et 130°C. Au bout de ce temps, le mélange réactionnel est versé sous agitation dans un bécher contenant 700 cm³ d'eau. Le précipité cristallin obtenu est filtré, lavé à l'eau et recristallisé dans le chloroforme après décoloration au charbon.

| | |
|---|---|
| Rendement | 19,5 g, 80% |
| F | 174°C |

(b) Variante

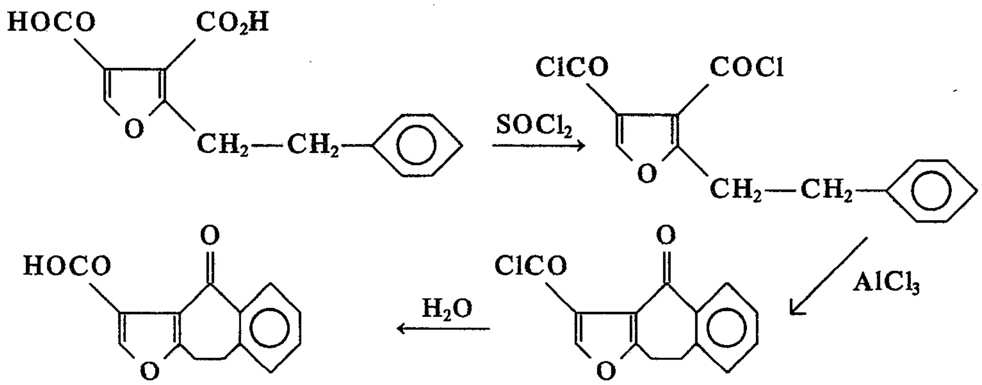


On porte au reflux pendant 3 heures un mélange de 0,25 M de phénéthyl-2 dicarboxy-3,4 furanne, 120 g de chlorure de thionyle. L'excès de chlorure de thionyle est éliminé sous vide, et le résidu de dichlorure d'acide furannique brut est dilué dans 250 cm$^3$ de sulfure de carbone.

Dans un ballon de 1 litre à trois tubulures, muni d'un agitateur mécanique, d'un réfrigérant et d'une ampoule à brome, on introduit 75 g de chlorure d'aluminium et 600 cm$^3$ de sulfure de carbone. Le mélange est porté au reflux pendant l'addition goutte à goutte de la solution sulfocarbonique de dichlorure d'acide furannique. On maintient le reflux pendant 2 heures après addition totale. Au bout de ce temps, le dégagement d'acide chlorhydrique est pratiquement terminé. Au repos, le mélange réactionnel se sépare en deux couches. La couche liquide est décantée, lavée à l'eau et séchée sur du sulfate de sodium. Après distillation du sulfure de carbone, on recueille environ 3 g d'acide cétonique attendu. La couche solide est décomposée par de la glace (1 kg), la réaction étant très vive et s'accompagnant de vapeurs de sulfure de carbone et d'acide chlorhydrique. Après décomposition, l'alumine est solubilisée par addition de 100 cm$^3$ d'acide chlorhydrique. Le précipité est filtré, lavé à l'eau et séché. On le recristallise dans le chloroforme après une décoloration au charbon.

On a rassemblé dans le tableau ci-dessous les caractéristiques du composé ainsi préparé ainsi que celles d'autres composés de formule (VIII) préparés de manière analogue.

| R | F°C | Rdt % | Formule brute | C % Calc. | C % tr. | H % Calc. | H % tr. |
|---|---|---|---|---|---|---|---|
| H | 174 | 85 | $C_{14}H_{10}O_4$ | 69,42 | 69,50 | 4,13 | 4,20 |
| $CH_3$ | 127 | 55 | $C_{15}H_{12}O_4$ | 70,31 | 70,00 | 4,68 | 4,40 |
| $C_2H_5$ | 107 | 50 | $C_{16}H_{14}O_4$ | 71,11 | 70,40 | 5,18 | 5,00 |

(4) Chlorocarbonyl-3 one-4 4H-benzo [4,5] cyclohepta [1,2-b]-furanne

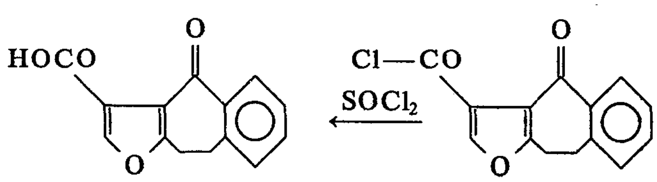


On porte au reflux pendant 4 heures, un mélange de 70 g de carboxy-3 one-4 4H-benzo [4,5] cyclohepta-[1,2-b]-furanne, 90 g de chlorure de thionyle et 110 cm$^3$ de toluène. Le solvant et le réactif en excès sont évaporés sous vide. Le résidu est repris à chaud avec 120 cm$^3$ de toluène, et décoloré sur charbon. On filtre. Le filtrat additionné de 30 cm$^3$ d'éther de pétrole est refroidi sur glace. On recueille un premier jet de 65 g de cristaux blanchâtres.

| | |
|---|---|
| Rendement | 86,3% |
| F | 92°C |

(5) Carbométhoxy-3 one-4 4H-benzo [4,5] cyclohepta [1,2-b]-furanne

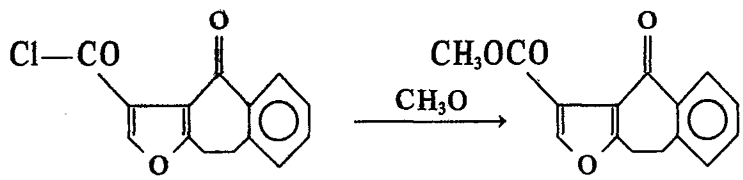


On porte au reflux pendant 15 heures un mélange de 0,1 M de chlorure d'acide cétonique et 200 $cm^3$ de méthanol. (La réaction peut être accélérée par addition de 15 $cm^3$ de pyridine). Le méthanol est ensuite distillé. Le résidu est additionné de 100 $cm^3$ de benzène, lavé à l'eau, à une solution saturée de bicarbonate de sodium, puis de nouveau à l'eau. Après séchage sur sulfate de sodium et élimination du benzène, on rectifie sous pression réduite.

| | |
|---|---|
| Rendement | 85 à 90% |
| E/16 mm Hg | 244° C |
| F | 53° C |

(6) Diéthylaminoéthylcarboxamido-3 diéthylamino-éthylamino-4-hydroxy-4 4H-benzo [4,5]-cyclohepta [1,2-b] furanne (composé n° 1)

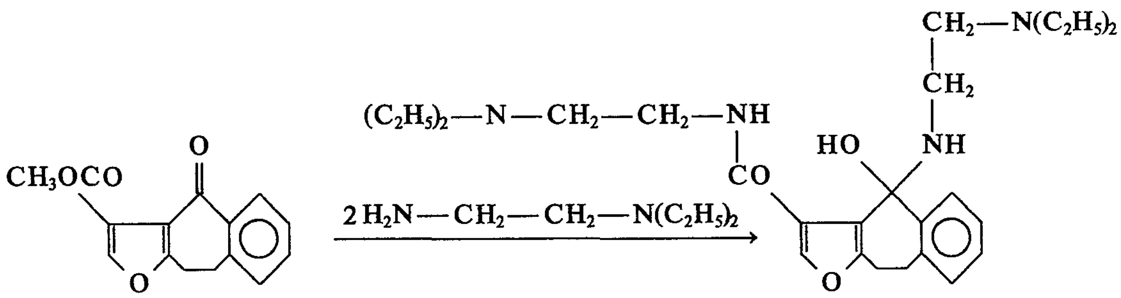


A 25,6 g d'ester cétonique (0,10 M), on ajoute 35 g de N-diéthyléthylènediamine commerciale. Le mélange est porté au reflux pendant 6 heures. L'excès d'amine est ensuite éliminé sous vide. Le résidu visqueux est repris par 120 $cm^3$ de benzène et décoloré au charbon. Le filtrat est refroidi, et additionné de 20 $cm^3$ d'éther de pétrole. On recueille 17,5 g de cristaux jaunâtres.

| | |
|---|---|
| Rendement | 40% |
| F | 133° C |

Analyse:
calculé pour $C_{26}H_{40}N_4O_3$
C 68,42 H 8,77 N 12,28
trouvé
C 68 H 8,36 N 12,00

Ce dérivé peut également être obtenu par réaction du chlorure d'acide avec l'amine en excès.

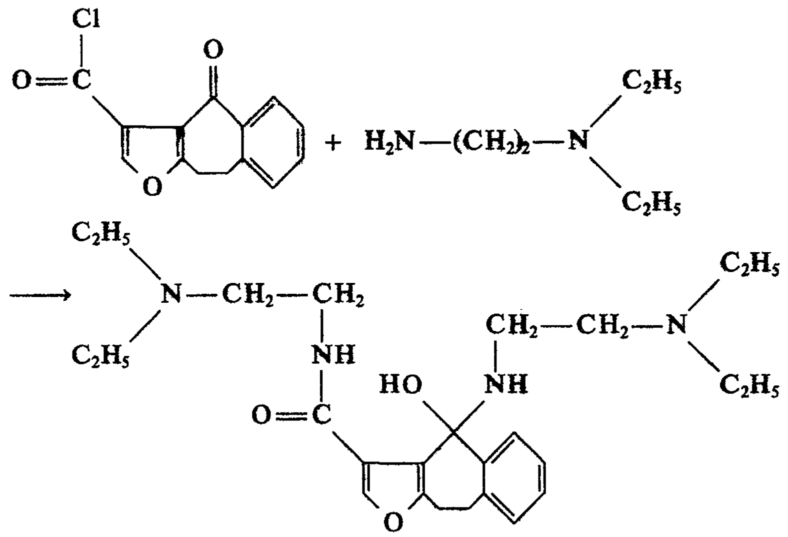

Dans un erlenmeyer, placé sous agitation magnétique, dans un bain d'eau froide, on verse 55 ml (0,39 mole) de N,N-diéthyléthylènediamine et on ajoute par petites fractions 20,3 g (0,078 mole) de chlorocarbonyl-3 one-4 4H-benzo-[4,5]-cyclohepta [1,2-b] furanne.

On agite 10 heures à température ambiante. Après réaction, on ajoute 100 ml d'éther éthylique, essore et lave à l'éther. Après séchage, on obtient 19,5 g de cristaux crèmes fondant à 125 – 130° C (rendement brut: 55%).

Ce produit brut est dissous dans 100 ml de chloroforme, passé sur alumine Merck activité I, amené à sec, et recristallisé dans l'acétone. On obtient 13,4 g de cristaux blancs fondant à 133° C (rendement: 37,5%).

$C_{26}H_{40}N_4O_3 = 456,6$.

Analyse:

| | | | |
|---|---|---|---|
| théorie | C 68,39 | H 8,33 | N 12,27 |
| trouvé | C 68,39 | H 8,81 | N 12,31. |

(7) Trichlorhydrate de diéthylamino-éthylcarboxamido-3 diéthylamino éthylamino-4 4H-benzo [4,5] cyclohepta [1,2-b]-furanne

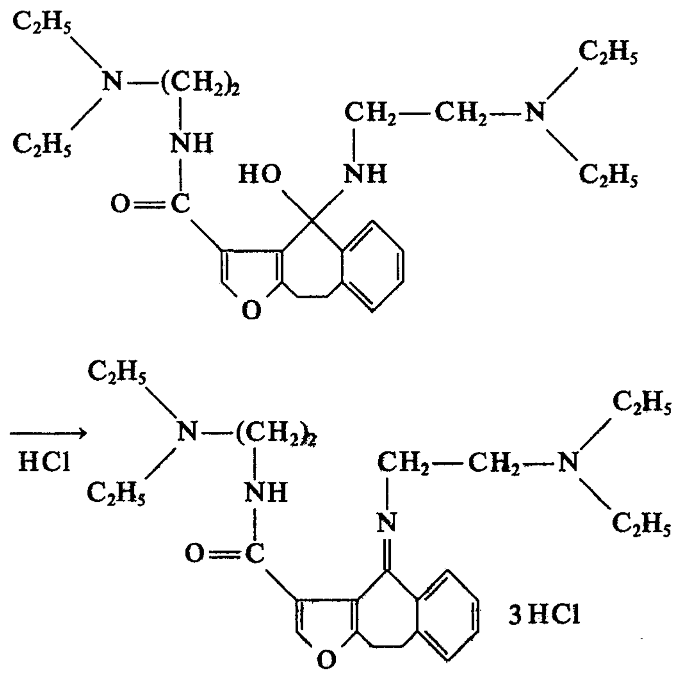


On agite à température ambiante pendant 4 heures, 4,56 g de diéthylamino-éthylcarboxamido-3 diéthylamino-éthylamino-4 hydroxy-4 4H benzo [4,5] cyclohepta [1,2-b] furanne (0,01 mole) et 50 ml

d'HCl 1N (0,05 mole). On obtient une solution limpide, jaune clair. Après réction, on chasse le solvant sous vide. On dissout à température ambiante l'huile obtenue dans 150 ml d'acétonitrile. On traite cette solution par 2 g de noir. On filtre. On chasse 100 ml de solvant sous vide et on abandonne 48 heures à 0° C.

On essore 3,36 g de cristaux crèmes (produit hygroscopique). (F = 173° C, rendement = 61,5%).

Analyse après séchage $C_{26}H_{33}N_4O_2 3\ HCl$ = 548

| | | | | |
|---|---|---|---|---|
| Théorie | C 56,97 | H 7,54 | N 10,22 | Cl 19,40 |
| Trouvé | C 56,85 | H 7,37 | N 10,20 | Cl 19,38 |

(8) Morpholino-éthyl-2 oxo-3 dihydro-2,3 benzo [6,7] cyclohepta [1,2,3-c,d]-furo [3,4-d] pyridazine (composé n° 2)

A 8 g de morpholino-éthylhydrazine dans 100 cm³ de n-propanol, on ajoute 12,2 g de céto-acide préparé en 3 (R = R' = H). Le mélange est porté au reflux pendant 4 heures; le propanol est évaporé sous pression réduite. Le résidu brun est soumis à l'extraction à chaud par 500 cm³ d'éther. L'éther est évaporé. Le résidu visqueux est recristallisé dans l'éthanol, après décoloration sur charbon.

| | |
|---|---|
| Rendement | 45,6% |
| Poids | 8 g |
| F | 135° C |

Analyse:
calculé pour $C_{20}H_{21}N_3O_3$
C 68,37 H 5,98 N 11,96
trouvé
C 68 H 5,8 N 11,6

(9) Oxo-3dihydro-2,3benzo[6,7]cyclohepta[1,2,3-c,d]-furo [3,4-d] pyridazine (R = H)

On porte au reflux pendant 10 heures un mélange de 2 g de céto-acide préparé en 3, 8 cm³ d'hydrazine hydratée et 40 cm³ d'acide acétique. La solution est concentrée à 10 cm³ sous vide, et versée dans un mélange de 30 cm³ d'acide chlorhydrique et de 100 cm³ d'eau. Le précipité est lavé à l'eau, séché et recristallisé dans le chloroforme. Les constantes des produits obtenus sont résumées dans le tableau suivant (R = H).

| Composé | R' | F°C | Formule brute | C % Calc. | C % tr. | H % Calc. | H % tr. | N % Calc. | N % tr. |
|---|---|---|---|---|---|---|---|---|---|
| 3 | H | 258 | $C_{14}H_{10}N_2O_2$ | 70,58 | 70,53 | 4,20 | 4,73 | 11,76 | 11,90 |
| 4 | $CH_3$ | 186 | $C_{15}H_{12}N_2O_2$ | 71,43 | 71,30 | 4,76 | 5,92 | 11,10 | 11,50 |
| 5 | $C_2H_5$ | 173 | $C_{16}H_{14}N_2O_2$ | 72,18 | 71,73 | 5,26 | 5,48 | 10,52 | 11,00 |

(10) a) Carboxamido- oxo-4 4H-benzo [4,5] cyclohepta-[1,2-b] furanne ($R = R' = R_1 = R_2 = H$) (composé n° 6)

A 25 g de chlorure d'acide brut obtenu en 4, refroidi sur glace, on ajoute avec précaution 200 cm³ d'ammoniaque préalablement refroidie. Le précipité est filtré, lavé à l'eau et séché. On le recristallise dans l'acide acétique. Rendement quantitatif. Cristaux blancs. F = 253° C.

Analyse:
calculé pour $C_{14}H_{11}NO_3$
C 69,71 H 4,56 N 5,81
trouvé
C 69,70 H 4,50 N 5,70

b) N-alkylcarboxamido-3 oxo-4 4H-benzo [4,5]-cyclohepta [1,2-b] furanne (R = R' = H) (composés n° 7, 8, 9)

Le chlorure d'acide est préparé à partir de 15 g de céto-acide (R = R' = H), et dilué dans 50 $cm^3$ de toluène. On l'ajoute goutte à goutte à une solution froide de 0,2 M d'amine dans 125 $cm^3$ de toluène. Après 30 minutes de reflux, le mélange est lavé à l'eau, séché sur sulfate de sodium, filtré et concentré sous pression réduite. Par addition d'éther, on obtient un précipité jaunâtre qui est recristallisé dans le tétrachlorure de carbone. Le rendement est quantitatif.

Les constantes des amides sont résumées dans le tableau suivant (R = R' = H) (composés n° 3, 4, 5)

| Composé | $R_1$, $R_2$ | F°C | Formule brute | C % Calc. | C % tr. | H % Calc. | H % tr. | N % Calc. | N % tr. |
|---|---|---|---|---|---|---|---|---|---|
| 7 | $(C_2H_5)_2$ | 118 | $C_{18}H_{19}NO_3$ | 72,72 | 71,80 | 6,40 | 6,40 | 4,71 | 4,60 |
| 8 | H,n · $C_3H_7$ | 112 | $C_{17}H_{17}NO_3$ | 72,08 | 71,20 | 6,00 | 5,90 | 4,94 | 4,90 |
| 9 | $(CH_2)_5$ | 147 | $C_{19}H_{19}NO_3$ | 73,78 | 72,95 | 6,15 | 6,20 | 4,53 | 4,00 |

On prépare d'autres composés du même type selon le mode opératoire suivant:

Dans un erlenmeyer à fond plat, surmonté d'un réfrigérant et d'une ampoule à brome, on introduit 3 M/100 de chlorure d'acide cétonique (R = R' = H), 30 $cm^3$ de tétrahydrofuranne anhydre. La solution est portée sur une plaque chauffante munie d'un agitateur magnétique. Par l'ampoule à brome, on ajoute un mélange de 3 M/100 de diamine et 10 $cm^3$ de tétrahydrofuranne; la précipitation du chlorhydrate est insrantanée. Après 5 minutes de reflux, on refroidit sur glace; la précipitation est complétée par addition de 10 $cm^3$ d'éther anhydre. On filtre et recristallise dans l'éthanol. Les rendements sont quantitatifs.

Les points de fusion sont déterminés sur un bloc de Maquenne.

Composé N° 10

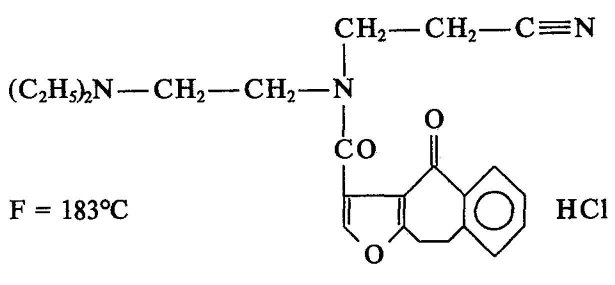

F = 183°C

Composé N° 11

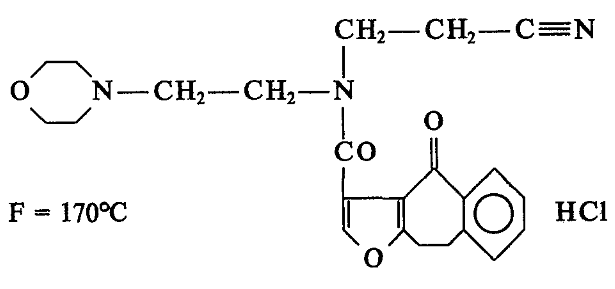

F = 170°C

Composé N° 12

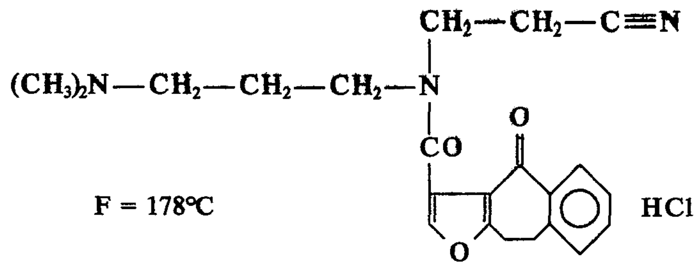

F = 178°C

Composé N° 13

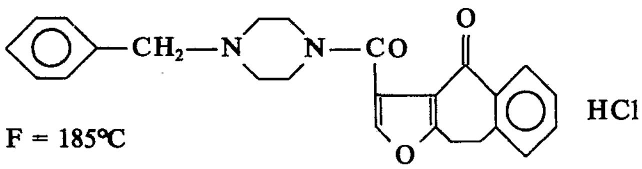

F = 185°C

(11) Pipéridinocarbonyl-3 hydroxy-4 4H-benzo [4,5] cyclohepta [1,2-b] furanne (composé n° 14)

On porte au reflux pendant 3 heures un mélange de 10 g du composé n° 9, 3 g de borohydrure de potassium, 60 $cm^3$ d'éthanol et 10 $cm^3$ d'eau. Les solvants sont ensuite évaporés sous vide. Le résidu, repris par l'eau, donne 9,5 g de cristaux blancs, recristallisables dans le toluène.

| | |
|---|---|
| Rendement | 95% |
| F | 158°C |

Analyse:
calculé pour $C_{19}H_{21}NO_3$
C 73,31  H 6,75  N 4,50
trouvé
C 72,60  H 6,60  N 4,45

Les composés selon la présente invention possèdent notamment une activité antifibrillante qui s'est avérée supérieure à celle du procaïnamide.

On rapporte ci-dessous des résultats d'essais toxicologiques et pharmacologiques.

Le tableau A résume les $DL_{50}$ par voie intrapéritonéale et par voie intraveineuse chez la souris.

Tableau A.

| Produits | DL 50 (mg/kg) | |
|---|---|---|
| | i.p. | i.v. |
| | Gomme arabique 10% | |
| Procaïnamide | 370 | 170 |
| Composé N° 1 | 90 | 24 |
| Composé N° 2 | >1000 | |
| Composé N° 9 | > 800 | |
| Composé N° 7 | > 800 | |
| Composé N° 3 | >1000 | |
| Composé N° 14 | 500 | |
| Composé N° 10 | | 26,7 |
| Composé N° 11 | | 316,7 |
| Composé N° 12 | | 116 |
| Composé N° 13 | | 20 |

Poûr les composés hydrosolubles, l'activité des composés selon l'invention a été comparée à celle du procaïnamide auquel on a attribué arbitrairement une activité 1, par deux tests classiques, le test de Dawes (Brit. J. Pharm. 1946 1 90—111) et la technique de Somani-Lum (Journal Pharm. Exp. Therap. 1966 147 194—204). Les résultats obtenus figurent dans le tableau B.

Tableau B

| Produits | Test de Dawes | Technique de Somani-Lum |
|---|---|---|
| Procaïnamide | 1 | 1 |
| Composé N° 1 | 13 | 20 |
| Composé N° 10 | 4 | 1 |
| Composé N° 11 | 4 | 5 |
| Composé N° 12 | 4 | 10 |
| Composé N° 13 | 13 | 20 |

Pour les autres composés, on a utilisé la technique de Lawson (J. Phar. Exp. Therap. 1968 160 22), les produits étant administrés par voie orale à l'état dissous dans la gomme arabique (à une concentration de 10%). On a attribué également au procaïnamide une activité égale à 1. Les résultats sont rassemblés dans le tableau C ci-dessous.

Tableau C

| Produits | Test de Lawson |
|---|---|
| Procaïnamide | 1 |
| Composé N° 1 | 12 |
| Composé N° 2 | 11 |
| Composé N° 9 | 10 |
| Composé N° 7 | 12 |
| Composé N° 3 | 12 |
| Composé N° 14 | 12 |

Il ressort de ces essais que les composés de l'invention possèdent un indice thérapeutique nettement plus favorable que celui du procaïnamide. De plus, les composés de l'invention se sont avérés présenter une durée d'action beaucoup plus longue que celle du procaïnamide.

Il découle de ce qui précède que les composés selon l'invention peuvent être utilisés en thérapeutique comme antifibrillant pour le traitement du système cardiovasculaire.

Ces composés sont administrés sous toute forme appropriée permettant leur administration par voie orale ou parentérale avec un dosage permettant une posologie de 0,05 à 20 mg/kg de patient par jour en une ou plusieurs prises.

**Revendications**

1.— Composés de formule:

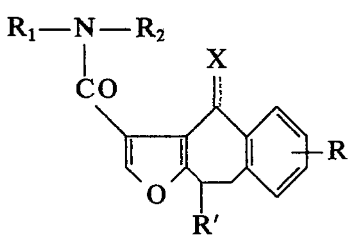

(I)

dans laquelle

R représente un atome d'hydrogène, un radical alcoyle ayant de 1 à 6 atomes de carbone ou un radical alkoxy ayant de 1 à 6 atomes de carbone,

R' représente un atome d'hydrogène, un radical alcoyle ayant de 1 à 6 atomes de carbone, un radical phényle ou un radical phénylalcoyle dont la partie alcoyle a de 1 à 6 atomes de carbone,

X est un radical hydroxy ou un radical oxo, et $R_1$ et $R_2$ pris séparément représentent indépendamment l'un de l'autre un atome d'hydrogène, un radical alcoyle ayant de 1 à 6 atomes de carbone portant éventuellement un radical cyano ou un radical de formule:

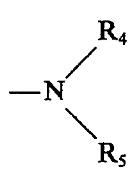

dans laquelle $R_4$ et $R_5$ représentent indépendamment l'un de l'autre un atome d'hydrogène, un radical alcoyle ayant de 1 à 6 atomes de carbone ou forment ensemble et avec l'atome d'azote sur lequel ils sont fixés un radical morpholino, et

$R_1$ et $R_2$, ensemble, forment avec l'atome d'azote sur lequel ils sont fixés, un radical morpholino, pipéridino ou pipérazino portant éventuellement sur le deuxième atome d'azote un radical alcoyle ayant de 1 à 6 atomes de carbone ou un radical phénylalcoyle dont la partie alcoyle a

de 1 à 6 atomes de carbone, ou bien

X est un reste aminal

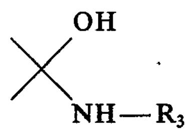

ou un radical de formule

$=N-R_3,$

$R_3$ représentant un atome d'hydrogène ou un radical alcoyle ayant de 1 à 6 atomes de carbone portant éventuellement un radical de formule:

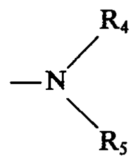

dans laquelle $R_4$ et $R_5$ ont la même signification que celle donnée précédemment, $R_1$ est identique à $R_3$ et $R_2$ est un atome d'hydrogène, ou bien

X forme avec $R_2$ un pont

$=N-$

et $R_1$ représente un atome d'hydrogène ou un radical alcoyle ayant de 1 à 6 atomes de carbone portant éventuellement un radical de formule:

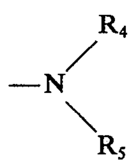

dans laquelle $R_4$ et $R_5$ ont la signification donnée précédemment,

et leurs sels d'addition avec des acides.

2. Composé selon la revendication 1, caractérisé par le fait que c'est le diéthylamino-éthylcarboxamido-3-diéthylamino éthylamino-4 hydroxy-4 4H-benzo [4,5]cyclohepta [1,2-b] furanne.

3. Composé selon la revendication 1, caractérisé par le fait que c'est la morpholino-éthyl-2 oxo-3 dihydro-2,3 benzo [6,7] cyclo-hepta [1,2,3-c, d] furo [3,4-d] pyridazine.

4. Composé selon la revendication 1, caractérisé par le fait que c'est l'oxo-3 dihydro-2,3 benzo [6,7] cyclohepta [1,2,3-c, d] furo [3,4-d] pyridazine.

5. Composé selon la revendication 1, caractérisé par le fait que c'est le N,N-diéthylcarboxamido-3 oxo-4 4H-benzo [4,5] cyclohepta [1,2-b] furanne.

6. Composé selon la revendication 1, caractérisé par le fait que c'est le pipéridinocarbonyl-3 oxo-4 4H-benzo [4,5] cyclohepta [1,2-b] furanne.

7. Composé selon la revendication 1, caractérisé par le fait que c'est le N-(morpholino-éthyl) N-(cyano-2 éthyl) carboxamido-3-oxo-4 4H-benzo [4,5] cyclohepta [1,2-b] furanne.

8. Composé selon la revendication 1, caractérisé par le fait que c'est le N-(diméthylamino-3 propyl) N-(cyano-2 éthyl)-carbox-amido-3 oxo-4 4H-benzo [4,5] cyclohepta [1,2-b] furanne.

9. Composé selon la revendication 1, caractérisé par le fait que c'est le N-(benzyl) pipérazinylcarbonyl-3 oxo-4 4H-benzo [4,5]-cyclohepta [1,2-b] furanne.

10. Composé selon la revendication 1, caractérisé par le fait que c'est le pipéridino-carbonyl-3 hydroxy-4 4H-benzo [4,5] cyclo-hepta [1,2-b] furanne.

11. Procédé de préparation de composés selon la revendication 1, caractérisé en ce que

(a) on effectue une condensation d'un (β-aryl)-alkyl-2-furanne de formule:

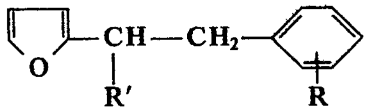

(II)

dans laquelle R et R' ont les significations données à la revendication 1, avec l'acétylène dicarboxylate de méthyle dans un solvant et l'on obtient un dérivé de l'oxa-7 bicyclo (2,2,1) heptadiène-2,5,

(b) on effectue une hydrogénation partielle de ce composé et l'on obtient un composé de formule:

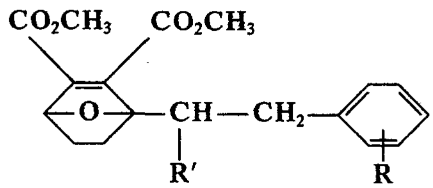

(IV)

(c) ce composé de formule (IV) est transformé directement selon une réaction d'Alder Rickert en (β-aryl) alkyl-2 dicarbométhoxy-3,4 furanne de formule:

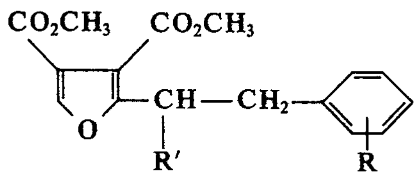

(V)

(d) on saponifie ce diester (V) et l'on obtient le diacide correspondant;

(e) on transforme le diacide en le dichlorure correspondant par action du chlorure de thionyle;

(f) on effectue une cyclisation intramoléculaire du dichlorure en solution dans le sulfure de carbone en présence de chlorure d'aluminium, l'on traite par l'eau le produit réactionnel et l'on obtient un céto-acide de formule:

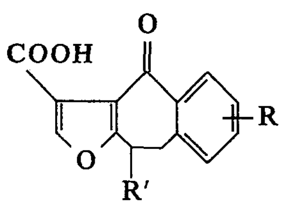

(VIII)

(g) on fait réagir le céto-acide (VIII) avec une hydrazine de formule $R'_1-NH-NH_2$, $R'_1$ ayant la signification donnée pour $R_1$ à la revendication 1 quand X forme avec $R_2$ un pont, et l'on obtient un composé de formule:

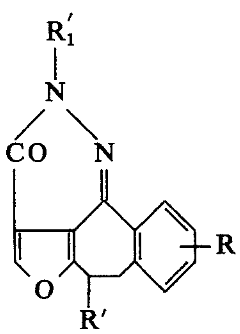

(Ia)

ou bien on fait réagir le céto-acide (VIII) avec le chlorure de thionyle ce qui conduit au chlorure d'acide (X) correspondant et l'on fait réagir ce chlorure d'acide avec une amine de formule:

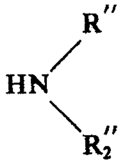

(XI)

dans laquelle $R_1''$ et $R_2''$ ont la signification donnée pour $R_1$ et $R_2$ à la revendication 1 quand X est un groupe oxo et l'on obtient un composé de formule:

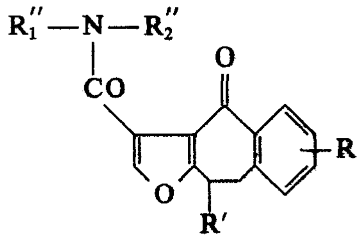

(Ib)

et éventuellement on réduit ce composé (I b) par $BH_4K$ et l'on obtient un composé de formule:

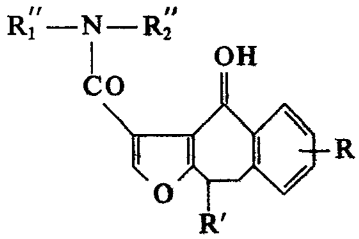

(Ib')

ou encore on fait réagir le chlorure d'acide (X) avec le méthanol ce qui conduit à l'ester correspondant, et l'on fait réagir cet ester avec une amine de formule:

$H_2N—R_1'''$

dans laquelle $R_1'''$ a la signification donnée pour $R_1$ quand X est un groupe $= N-R_3$ ou un reste aminal

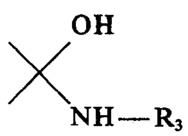

et l'on obtient le composé de formule:

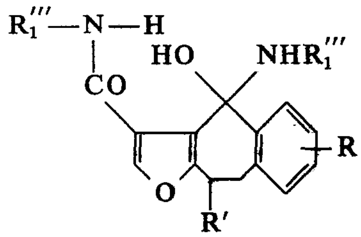

(Ic')

ou encore on fait réagir le chlorure d'acide de formule (X) avec l'amine de formule $H_2NR_1'''$ obtenant ainsi directement le composé de formule (I c'), et éventuellement on déshydrate le composé de formule (I c') obtenant ainsi le composé de formule:

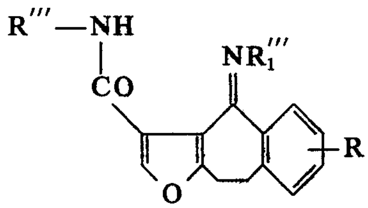

(Ic)

**12. — Variante du procédé selon la revendication 3, caractérisée en ce que l'on obtient directement le céto-acide (VIII) par cyclisation directe du diacide obtenu au stade (d) dans le l'acide polyphosphorique entre 100 et 130°C.**

**13. — Application des composés selon la revendication 1 ou l'une des revendications 2 à 10 en tant que principes actifs de médicaments ayant notamment une activité antifibrillante.**

**14. — Composés de formule:**

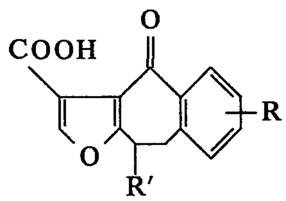

convenant comme intermédiaires dans le procédé selon la revendication 3, caractérisé en ce que R représente un atome d'hydrogène, un radical alcoyle ayant de 1 à 6 atomes de carbone ou un radical alkoxy ayant de 1 à 6 atomes de carbone,

R' représente un atome d'hydrogène, un radical alcoyle ayant de 1 à 6 atomes de carbone, un radical phényle ou un radical phénylalcoyle dont la partie alcoyle a de 1 à 6 atomes de carbone, leurs esters et chlorures d'acides.

**Patentansprüche**

1. Verbindungen der Formel:

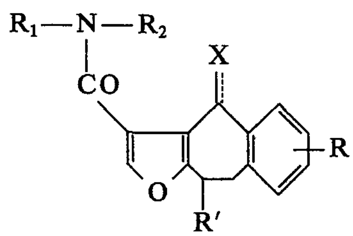

(I)

worin bedeuten:

R ein Wasserstoffatom, eine Alkylgruppe mit 1 bis 6 Kohlenstoffatomen oder eine Alkoxygruppe mit 1 bis 6 Kohlenstoffatomen,

R' ein Wasserstoffatom, eine Alkylgruppe mit 1 bis 6 Kohlenstoffatomen, eine Phenylgruppe oder eine Phenylalkylgruppe, deren Alkylrest 1 bis 6 Kohlenstoffatome aufweist,

X eine Hydroxygruppe oder eine Oxygruppe, und

$R_1$ und $R_2$ jeweils für sich unabhängig voneinander ein Wasserstoffatom, eine Alkylgruppe mit 1 bis 6 Kohlenstoffatomen, die gegebenenfalls einen Cyanorest oder eine Gruppe der Formel trägt:

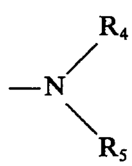

worin $R_4$ und $R_5$ unabhängig voneinander ein Wasserstoffatom oder eine Alkylgruppe mit 1 bis 6 Kohlenstoffatomen darstellen oder zusammen mit dem Stickstoffatom, an welches sie gebunden sind, einen Morpholinorest bilden, und

$R_1$ und $R_2$ zusammen mit dem Stickstoffatom, an welches sie gebunden sind, einen Morpholinorest, Piperidinorest oder Piperazinorest bilden, die gegebenenfalls an dem zweiten Stickstoffatom einen Alkylrest mit 1 bis 6 Kohlenstoffatomen oder einem Phenylalkylrest, dessen Alkylrest 1 bis 6 Kohlenstoffatome aufweist, tragen, oder auch

X eine Aminalgruppe

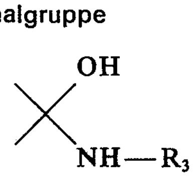

oder eine Gruppe der Formel

$=N-R_3$,

worin $R_3$ ein Wasserstoffatom oder einen Alkylrest mit 1 bis 6 Kohlenstoffatomen darstellt, der gegebenenfalls einen Rest der folgenden Formel tragen kann:

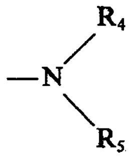

worin $R_4$ und $R_5$ die zuvor angegebene Bedeutung besitzen, $R_1$ mit $R_3$ identisch ist und $R_2$ ein Wasserstoffatom darstellt, oder auch

X mit $R_2$ eine Brücke

$=N-$

bildet und $R_1$ ein Wasserstoffatom oder einen Alkylrest mit 1 bis 6 Kohlenstoffatomen darstellt, der gegebenenfalls einen Rest der folgenden Formel tragen kann:

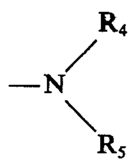

worin $R_4$ und $R_5$ die zuvor angegebene Bedeutung besitzen,

sowie deren Additionssalze mit Säuren.

2. Verbindung nach Anspruch 1, dadurch gekennzeichnet, daß sie Diäthylamino-ethylcarboxamido-3-diethylamino-ethylamino-4-hydroxy-4,4H-benzo[4,5]cyclohepta[1,2-b]furan ist.
3. Verbindung nach Anspruch 1, dadurch gekennzeichnet, daß sie Morpholino-ethyl-2-oxo-3-dihydro-2,3-benzo[6,7]cyclohepta[1,2,3-c,d]furo[3,4-d]pyridazin ist.
4. Verbindung nach Anspruch 1, dadurch gekennzeichnet, daß sie Oxo-3-dihydro-2,3-benzo[6,7]cyclohepta[1,2,3-c,d]-furo[3,4-d]pyridazin ist.
5. Verbindung nach Anspruch 1, dadurch gekennzeichnet, daß sie N,N-Diethylcarboxamido-3-oxo-4,4H-benzo[4,5]cyclohepta[1,2-b]furan ist.
6. Verbindung nach Anspruch 1, dadurch gekennzeichnet, daß sie Piperidinocarbonyl-3-oxo-4,4H-benzo[4,5]cyclohepta[1,2-b]furan ist.
7. Verbindung nach Anspruch 1, dadurch gekennzeichnet, daß sie N-(Morpholino-ethyl)-N-(cyano-2-ethyl)-carboxamido-3-oxo-4,4H-benzo[4,5]cyclohepta[1,2-b]furan ist.
8. Verbindung nach Anspruch 1, dadurch gekennzeichnet, daß sie N-(Dimethylamino-3-propyl)-N-(cyano-2-ethyl)-carboxamido-3-oxo-4,4H-benzo[4,5]cyclohepta[1,2-b]furan ist.
9. Verbindung nach Anspruch 1, dadurch gekennzeichnet, daß sie N-(Benzyl)-piperazinylcarbonyl-3-oxo-4,4H-benzo[4,5]cyclohepta[1,2-b]furan ist.
10. Verbindung nach Anspruch 1, dadurch gekennzeichnet, daß sie Piperidino-carbonyl-3-hydroxy-4,4H-benzo[4,5]cyclohepta[1,2-b]furan ist.
11. Verfahren zur Herstellung von Verbindungen nach Anspruch 1, dadurch gekennzeichnet, daß man:

(a) eine Kondensation eines ($\beta$-Aryl)-alkyl-2-furans der Formel:

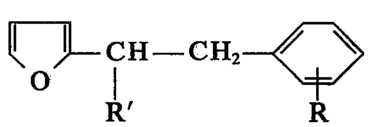

(II)

**worin R und R' die in Anspruch 1 angegebenen Bedeutungen besitzen, mit Acetylenmethyldicarboxylat in einem Lösungsmittel kondensiert und ein Derivat von Oxa-7-bicyclo[2,2,1]heptadien-(2,5) gewinnt,**

(b) eine partielle Hydrierung dieser Verbindung durchführt und eine Verbindung der Formel

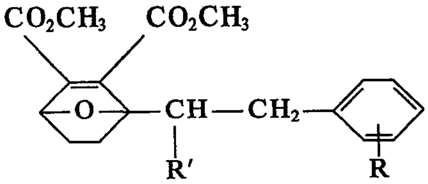

(IV)

erhält,

(c) diese Verbindung der Formel (IV) direkt in einer Alder-Rickert-Reaktion in ($\beta$-Aryl)-alkyl-2-dicarbomethoxy-3,4-furan der Formel:

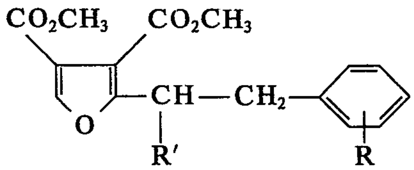

(V)

umwandelt,

(d) diesen Diester (V) verseift und die entsprechende Disäure erhält,

(e) die Disäure in das entsprechende Dichlorid durch Einwirkung von Thionylchlorid überführt,

(f) einen intramolekularen Ringschluß des Dichlorids in Schwefelkohlenstofflösung in Anwesenheit von Aluminiumchlorid durchführt, das Reaktionsprodukt mit Wasser behandelt und eine Ketosäure der Formel

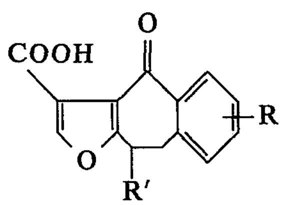

(VIII)

erhält,

(g) diese Ketosäure (VIII) mit einem Hydrazin der Formel $R_1'-NH-NH_2$, wobei $R_1'$ die für $R_1$ in Anspruch 1 angegebene Bedeutung besitzt, wenn X mit $R_2$ eine Brücke bildet, reagieren läßt und eine Verbindung der Formel

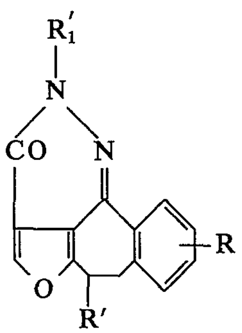

(Ia)

erhält, oder auch die Ketosäure (VIII) mit Thionylchlorid reagieren läßt, was zum entsprechenden Säurechlorid (X) führt, und man dieses Säurechlorid mit einem Amin der Formel:

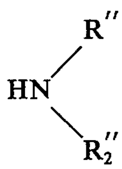

(XI)

worin $R_1''$ und $R_2''$ die für $R_1$ und $R_2$ in Anspruch 1 angegebene Bedeutung besitzen, wenn X eine Oxogruppe ist, reagieren läßt, und eine Verbindung der Formel:

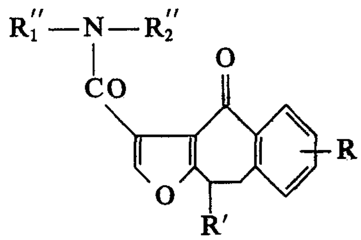

(Ib)

erhält, und gegebenenfalls diese Verbindung (I b) mittels $BH_4K$ reduziert und eine Verbindung der Formel

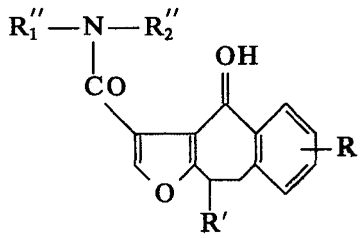

(Ib')

erhält, oder auch das Säurechlorid (X) mit Methanol reagieren läßt, was zu dem entsprechenden Ester führt, und diesen Ester mit einem Amin der Formel:

$H_2N—R_1'''$

reagieren läßt, worin $R_1'''$ die für $R_1$ angegebene Bedeutung besitzt, wenn X die Gruppe $=N-R_3$ oder einen Aminalrest

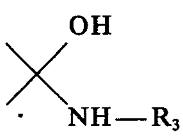

bedeutet, und man die Verbindung der Formel

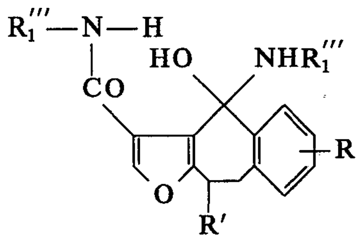

(Ic')

erhält oder auch das Säurechlorid der Formel (X) mit einem Amin der Formel $H_2NR_1'''$ reagieren läßt, wobei man auf diese Weise direkt die Verbindung der Formel (I c') erhält, und gegebenenfalls die Verbindung der Formel (I c') dehydratisiert, wobei auf diese Weise die Verbindung der Formel:

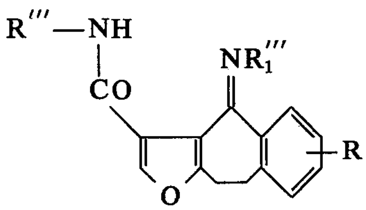

(Ic)

erhalten wird.

12. Abwandlung des Verfahrens nach Anspruch 3, dadurch gekennzeichnet, daß man die Ketosäure (VIII) direkt durch direkten Ringschluß der in der Stufe (d) erhaltenen Disäure in Polyphosphorsäure zwischen 100 und 130° C erhält.

13. Anwendung der Verbindung nach Anspruch 1 oder einer der Ansprüche 2 bis 10 als Aktivwirkstoff in Arzneimitteln mit insbesondere einer antifibrillären Aktivität.

14. Verbindungen der Formel:

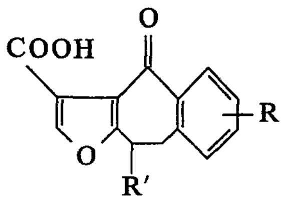

geeignet als Zwischenprodukte in dem Verfahren nach Anspruch 3, dadurch gekennzeichnet, daß hierin bedeuten:

R ein Wasserstoffatom, eine Alkylgruppe mit 1 bis 6 Kohlenstoffatomen oder eine Alkoxygruppe mit 1 bis 6 Kohlenstoffatomen,
R' ein Wasserstoffatom, eine Alkylgruppe mit 1 bis 6 Kohlenstoffatomen, eine Phenylgruppe oder eine Phenylalkylgruppe, deren Alkylrest 1 bis 6 Kohlenstoffatome aufweist,

sowie deren Ester und Säurechloride.

## Claims

1. Compounds of the following formula:

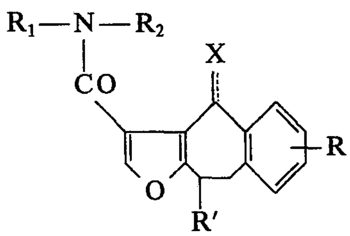

(I)

in which

R represents a hydrogen atom, an alkyl radical having from 1 to 6 carbon atoms or an alkoxy radical with from 1 to 6 carbon atoms;
R' represents a hydrogen atom, an alkyl radical having from 1 to 6 carbon aroms, a phenyl radical or a phenylalkyl radical the alkyl part of which has from 1 to 6 carbon atoms,
X is a hydroxy radical or an oxo radical, and
$R_1$ and $R_2$ taken separately represent independently from one another a hydrogen atom, an alkyl radical having from 1 to 6 carbon atoms, carrying possibly a cyano radical or a radical of the following formula:

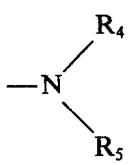

in which $R_4$ and $R_5$ represent independently of one another a hydrogen atom, an alkyl radical having from 1 to 6 carbon atoms or form together and with the nitrogen atom to which they are fixed a morpholino radical, and
$R_1$ and $R_2$ together form with the nitrogen atom to which they are fixed a morpholino, piperidino or piperazino radical carrying possibly on the second nitrogen atom an alkyl radical having from 1 to 6 carbon atoms or a phenylalkyl radical the alkyl part of which has from 1 to 6 carbon atoms, or
X is an aminal rest

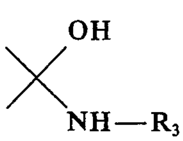

or a radical of formula

$=N-R_3$,

$R_3$ representing a hydrogen atom or an alkyl radical having from 1 to 6 carbon atoms, possibly carrying a radical of the following formula:

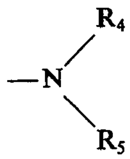

in which $R_4$ and $R_5$ have the same meaning as that given above, $R_1$ is identical to $R_3$, and $R_2$ is a hydrogen atom, or

X forms with $R_2$ a bridge

$=N-$,

and $R_1$ represents a hydrogen atom or an alkyl radical having from 1 to 6 carbon atoms, possibly carrying a radical of the following formula:

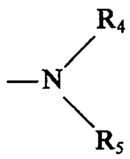

in which R and $R_5$ have the same meaning as above,

and their addition salts with acids.

2. A compound as claimed in claim 1, characterized in that it is diethylamino-3-ethylcarboxamido-diethylamino-4-ethylamino-4-hydroxy[4,5]-4H-benzo-[1,2-b]-cyclohepta-furane.
3. A compound as claimed in claim 1, characterized in that it is 2-morpholino-thyl-3-oxo2,3-dihydro-[6,7]-benzo-[1,2,3-c,d]-cyclohepta-[3,4-d]-furo-pyridazine.
4. A compound as claimed in claim 1, characterized in that it is 3-oxo-2,3-dihydro-[6,7]-benzo-[1,2,3-c,d]-cyclohepta-[3,4-d]-furo-pyridazine.
5. A compound as claimed in claim 1, characterized in that it is N,N-3-diethylcarboxamido-4-oxo-[4,5]-4H-benzo-[1,2-b]-cyclohepta-furane.
6. A compound as claimed in claim 1, characterized in that it is 3-piperidinocarbonyl-4-oxo-[4,5]-4H-benzo-[1,2-b]-cycloheptafurane.
7. A compound as claimed in claim 1, characterized in that it is N-(morpholino-ethyl)-N-(2-cyanoethyl)-3-carboxamido-4-oxo-[4,5]-4H-benzo-[1,2-b]-cyclohepta-furane.
8. A compound as claimed in claim 1, characterized in that it is N-(3-dimethylamino propyl)-N-(2-cyano ethyl)-3-carboxamido-4-oxo-[4,5]-4H-benzo-[1,2-b]-cyclohepta-furane.
9. A compound as claimed in claim 1, characterized in that it is 3-N-(benzyl)-piperazinylcarbonyl-4-oxo-[4,5]-4H-benzo-[1,2-b]-cyclohepta-furane, or.
10. A compound as claimed in claim 1, characterized in that it is 3-piperidino-carbonyl-4-hydroxy-[4,5]-4H-benzo-[1,2-b]-cyclohepta-furane.
11. A process for preparation of compounds as claimed in claim 1, characterized by the following steps:

(a) condensation of a ($\beta$-aryl)-2-alkyl-furane of the following formula:

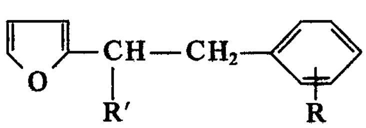

(II)

in which R and R' have the same meaning as in claim 1, with methyl dicarboxylate acetylene in a solvent, and obtaining a derivative of 7-oxa-[2,2,1]-bicyclo-2,5-heptadiene,

(b) partly hydrogenating this compound and obtaining a compound of the following formula:

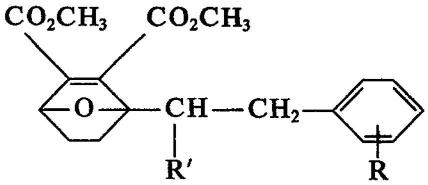

(IV)

(c) such compound of formula (IV) is directly converted in accordance with an Alder Rickert's reaction into ($\beta$-aryl)-2-alkyl-3,4-dicarbomethoxy-furane of the following formula:

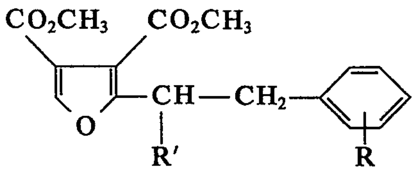

(V)

(d) saponification of this diester (V) and obtaining the corresponding diacid;

(e) converting the diacid into the corresponding dichloride by the action of the thionyl chloride;

(f) effecting an intramolecular cyclization of the dichloride in solution in the carbon sulfide in the presence of aluminum chloride, treating in water the reaction product and obtaining a keto-acid of the following formula:

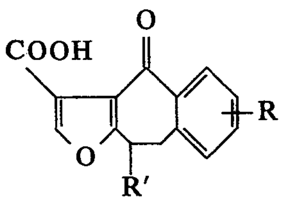

(VIII)

(g) reacting the keto-acid (VIII) with a hydrazine of formula $R'_1-NH-NH_2$, $R'_1$ having the meaning given for $R_1$ in claim 1, when X forms with $R_2$ a bridge, and obtaining a compound of the following formula:

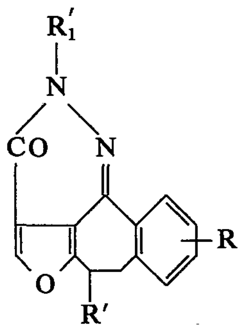

(Ia)

or reacting the keto-acid (VIII) with the thionyl chloride which leads to the corresponding acid chloride (X) and reacting such acid chloride with an amine of the following formula:

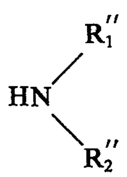

(XI)

in which $R''_1$ and $R''_2$ have the meaning as given above for $R_1$ and $R_2$ in claim 1, when X is an oxo group and obtaining a compound of the following formula:

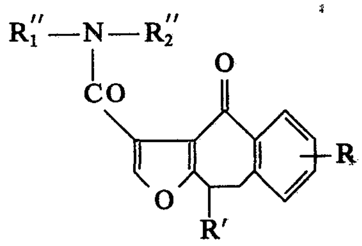

(Ib)

and possibly, reducing such compound (I b) by $BH_4K$, and obtaining a compound of the following formula:

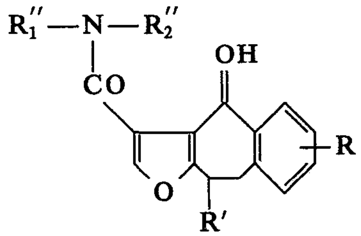

(Ib′)

or else, reacting the acid chloride (X) with methanol which leads to the corresponding ester, and reacting such ester with an amine of formula:

$H_2N-R_1'''$

in which $R_1'''$ has the same meaning as given above for $R_1$ when X is a group $=N-R_3$ or an aminal rest

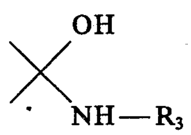

and obtaining the compound of the following formula:

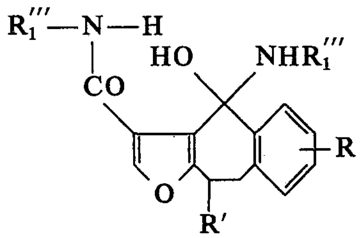

(Ic′)

or else, reacting the acid chloride of formula (X) with the amine of formula $H_2NR_1'''$, thereby directly obtaining the compound of formula (I c′), and possibly, dehydrating the compound of formula (I c′) thereby to obtain the compound of the following formula:

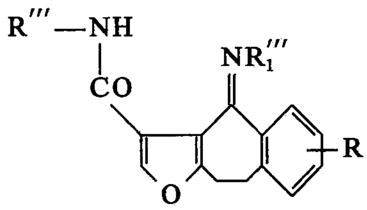

(Ic)

12. An alternative embodiment of the process as claimed in claim 11, characterized in that the keto-acid (VIII) is directly obtained by direct cyclization of the diacid obtained in stage (d) in polyphosphoric acid between 100 and 130° C.

13. An application of the compounds as claimed in claim 1 or one of claims 2 to 10 as the active principles of medicaments having in particular antifibrillating activity.

14. Compounds of the following formula:

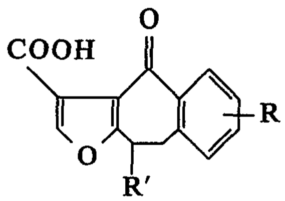

appropriate as intermediary products in the process as claimed in claim 11, wherein

R represents a hydrogen atom, an alkyl radical having from 1 to 6 carbon or an alkoxy radical having from 1 to 6 carbon atoms,

R' represents a hydrogen atom, an alkyl radical having from 1 to 6 carbon atoms, a phenyl radical or a phenylalkyl radical the alkyl part of which has from 1 to 6 carbon atoms,

their esters and acid chlorides.